# EUROPEAN PATENT APPLICATION

(11) **EP 4 813 466 A1**
(43) Date of publication of application: **30.09.2026**
(21) Application number: 25382286.0
(22) Date of filing: 26.03.2025
(51) Int. Cl.: A61Q 19/10, A61K 8/37, A61K 8/46

(54) **COMPOSITION COMPRISING AN ACYL LACTYLATE AND A MIXTURE OF AMPHOTERIC SURFACTANTS COMPRISING AN ALKYL OR ALKENYL HYDROXYSULTAINE AND AN ALKYL- OR ALKENYLAMIDOPROPYL HYDROXYSULTAINE**

(71) Applicant: KAO CORPORATION, S.A., 08210 Barberà del Vallès, Barcelona (ES)
(72) Inventor: SUBIRATS VICIENT, Neus, E-08210 Barberà del Vallès (Barcelona) (ES); MARIMON MARGARIT, Núria, E-08210 Barberà del Vallès (Barcelona) (ES)
(74) Representative: ABG Intellectual Property Law, S.L.

(57) **Abstract**

The present invention relates to a composition comprising an acyl lactylate and a mixture of amphoteric surfactants comprising an alkyl or alkenyl hydroxysultaine and an alkyl or alkenyl amidopropyl hydroxysultaine, to a method for the preparation of said composition, the cosmetic composition comprising said composition and the use of said cosmetic composition in hair and/or skin cleansing applications.

## Description

### FIELD OF THE INVENTION

The present invention relates to a composition comprising an acyl lactylate and a mixture of amphoteric surfactants comprising an alkyl or alkenyl hydroxysultaine and an alkyl- or alkenylamidopropyl hydroxysultaine, to a method for the preparation of said composition, and the use of said composition in hair and/or skin cleansing applications.

### STATE OF THE ART

Skin and hair cleanser compositions are required to have not only good detergency and cleansing ability, but they also need to be mild and well tolerated by the skin and/or hair and not to cause excessive defatting or dryness to the skin and hair, with reduced tackiness, homogeneous, to be of easy applicability and to exhibit good foaming properties, such as foam stability, foam quantity, foam quality and foam smoothness. In addition, skin and hair cleanser compositions are demanded by consumers to exhibit good viscosity as well as good rheological properties of stability, feel and flow. Also, there is a concern in the customer products market towards the sustainability of cosmetic products. Thus, there is also a requirement to formulate products with ingredients that are considered "green" or "natural" since they are derived from renewable and/or sustainable sources.

The use of anionic surfactants in skin and/or hair cleansers is well known by the person skilled in the art. Sulfated anionic surfactants, including anionic sulfates and anionic sulfonates, are well known for providing a high volume of stable foam, as well as being efficient skin and/or hair cleaners. Accordingly, sulfated anionic surfactants have been the primary surfactant used in skin and/or hair cleansing products. However, sulfated anionic surfactants have disadvantages such as stripping the hair of natural oils that condition the hair, thus giving the hair a dry feel. Sulfated anionic surfactants are also harsh to the skin and eyes.

Anionic lactylate surfactants are an alternative to sulfate-containing cosmetic compositions. They are commonly used in the cosmetic and personal care industries.

Lactylate surfactants, particularly sodium lauroyl lactylates, can be used as both primary and secondary surfactants in cosmetic compositions. However, one of the drawbacks of the acyl lactylate is its physical form. Sodium lauroyl lactylate can be found as an opaque, thick paste, alternatively it can be found in the market as a semi solid component, which cannot be directly poured by the formulator into a reactor to prepare the cosmetic composition, unless heated. This solid or semi solid state difficults its incorporation in formulations, being necessary to heat the product at high temperatures.

The preparation of compositions comprising such lactylate surfactants requires a step of heating, thus generating a high energy consumption, it can imply a specific addition order to the formulation and it can also limit the compatibility with certain ingredients that are not stable at high temperatures.

For the cosmetic compositions comprising the surfactant mixture, the obtaining of more efficient processes, wherein less heating is required, or residual heat is used, is a benefit, as a more energy efficient process can lead to not only a reduction in costs but also in a significant lower amount of CO₂ gas emissions.

As personal care consumers worldwide are more concerned in the need of eco-friendly solutions, a cold process, thus, not requiring high temperatures to prepare the cosmetic composition, is needed, reducing both the energy demand and manufacturing time required. Moreover, significantly less water is used in those processes at lower temperature than those requiring high temperatures, thus relying in water for a cooling function. Thus, personal care and cosmetic formulations prepared using cold processes can have lower carbon and lower water footprints. To summarize, a cold-processable product comprising a lactylate surfactant has several benefits for the manufacturer of cosmetic formulations, including the costs savings due to the reduction in the energy consumption and the production time, the improved safety of production processes because of the avoidance of high temperatures, and the no limitation on the use of heat sensitive ingredients in the formulations.

US5911981 describes a surfactant blend comprising acyl lactylate and a nonionic surfactant or an amphoteric surfactant. This patent describes surfactant blends for cosmetic applications generating a stable foam.

EP0562720 describes a cleansing composition comprising C6 to C16 acyl lactylates and one or more co-surfactants, such as acyl taurates, isethionates, sarcosinates, sulfosuccinates, phosphates, betaines, sultaines, amphocarboxylates or alkylpolyglycosides. This patent application describes cosmetic cleansing compositions with good foaming power.

From the state of the art set forth above, it can be seen that there is still a need for further compositions comprising an acyl lactylate for cosmetic compositions, particularly for skin and/or hair cleansing.

### SUMMARY OF THE INVENTION

The present invention aims at addressing the above-described needs and provides a composition comprising an acyl lactylate and a mixture of amphoteric surfactants comprising at least an alkyl or alkenyl hydroxysultaine and at least an alkyl- or alkenylamidopropyl hydroxysultaine, a process for the preparation of said composition; and its use to prepare cosmetic compositions for skin and/or hair cleansing. The cosmetic compositions prepared with the compositions of the invention are able to comply with the requirements imposed on them and provide good performance. In particular, they exhibit good viscosity, good cloud point, good rheological properties of stability, feel and flow, good foaming properties, such as foam stability, speed of foam formation, foam quantity, foam quality and foam smoothness. The compositions do also have good cleansing ability, they are mild and are well tolerated by the skin and/or hair and do not cause excessive defatting or dryness to the skin and/or hair, they also have reduced tackiness, they are homogenous and cold processable.

Thus, the first aspect of the present invention is related to a composition comprising:
a) at least an acyl lactylate, and
b) a mixture of amphoteric surfactants comprising
   b1) at least an alkyl or alkenyl hydroxysultaine
   b2) at least an alkyl- or alkenylamidopropyl hydroxysultaine.

Processes for the preparation of the composition according to the first object are also an object of the invention.

Another aspect of the present invention is the use of the composition of the invention to prepare cosmetic compositions for the care of skin and/or hair, in particular for cleansing skin and/or hair.

Another aspect of the present invention is the use of the composition of the invention for the care of skin and/or hair, in particular for cleansing skin and/or hair.

It is also an aspect of the present invention a process of preparation of the cosmetic composition. The use of the composition according to the invention for the cleansing treatment of skin and/or hair and a method of cleansing skin and/or hair with the composition are also aspects of the present invention.

### DETAILED DESCRIPTION OF THE INVENTION

All percentage are weight percentages, unless otherwise indicated.

As "cosmetic composition" it is understood a composition suitable as an ingredient to be used in cosmetic and personal care applications, in particular for skin and/or hair care.

The main object of the present invention is a composition comprising:
a) at least an acyl lactylate, and
b) a mixture of amphoteric surfactants comprising
   b1) at least an alkyl or alkenyl hydroxysultaine
   b2) at least an alkyl or alkenyl amidopropyl hydroxysultaine.

### Acyl lactylate (a)

The present invention comprises at least an acyl lactylate. The term "acyl lactylate" refers to a salt of an ester formed from the OH group of lactic acid and an acyl group. Said ester may contain one or more, preferably 1 to 3 lactic acid moieties, i.e. -O-CH(CH₃)-C(=O)-, which have been covalently bond to each other forming ester groups and one of said moieties being bound to the acyl group by the oxygen atom bond to the -CH(CH₃)- moiety, and the same one (in case of 1 lactic acid moiety) or another forming the salt with the carboxylate group -COO⁻.

Preferably, the acyl lactylate is represented by formula (I) 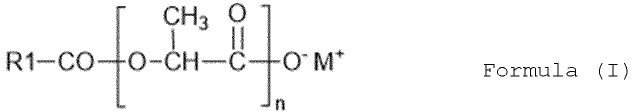 wherein R1 is a linear or branched alkyl or alkenyl group containing from 5 to 23 carbon atoms, preferably from 7 to 17 carbon atoms, more preferably from 9 to 15 carbon atoms, and from 0 to 3 double bonds; n is a number from 1 to 3, preferably 1 or 2; and M is selected from the group consisting of an alkali metal, an alkaline earth metal and ammonium; preferably an alkali metal.

As used in the present document, the term "alkyl" refers to a straight or branched hydrocarbon chain radical consisting of carbon and hydrogen atoms, containing no unsaturation, having the indicated number of carbon atoms, and which is attached to the rest of the molecule by a single bond.

As used in the present document, the term "alkenyl" refers to a linear hydrocarbon chain radical consisting of the indicated number of carbon atoms and containing at least one double bond, preferably 1, 2, or 3 double bonds, having the indicated number of carbon atoms (which is at least two), and which is attached to the rest of the molecule by a single bond.

As used in the present document, the term "alkali metal" refers to an element from group 1 of the periodic table, preferably sodium and potassium, still more preferably sodium.

As used in the present document, the term "alkaline earth metal" refers to an element from group 2 of the periodic table, preferably magnesium and calcium.

As used in the present document, the term "ammonium" refers to NH₄⁺.

Examples of acyl lactylates represented by formula (I) include sodium lauroyl monolactylate, sodium lauroyl dilactylate, sodium myristoyl monolactylate, sodium myristoyl dilactylate, sodium decanoyl monolactylate, sodium decanoyl dilactylate, sodium behenoyl monolactylate, sodium behenoyl dilactylate, sodium oleoyl monolactylate, sodium oleoyl dilactylate, sodium caproyl monolactylate, sodium caproyl dilactylate, sodium cocoyl monolactylate, sodium cocoyl dilactylate, potassium lauroyl monolactylate, potassim lauroyl dilactylate, triethanolammonium lauroyl monolactylate, triethanolammonium lauroyl dilactylate, or mixtures thereof.

In one embodiment, R1 is a linear or branched alkyl group containing from 5 to 23 carbon atoms, preferably from 7 to 17 carbon atoms, more preferably from 9 to 15 carbon atoms.

In one embodiment, R1 is a linear alkyl group containing from 5 to 23 carbon atoms, preferably from 7 to 17 carbon atoms, more preferably from 9 to 15 carbon atoms.

In one embodiment, n is selected from 1, 2 and 3; preferably from 1 and 2.

Preferably, the acyl lactylates represented by formula (I) are selected from the group consisting of sodium lauroyl monolactylate, sodium lauroyl dilactylate, sodium myristoyl monolactylate, sodium myristoyl dilactylate, and mixtures thereof; more preferably sodium lauroyl monolactylate, sodium lauroyl dilactylate, and mixtures thereof.

Commercial acyl lactylates typically are a blend of the monolactylate (n=1) and the dilactylate (n=2) and might contain small amounts of trilactylate (n=3). As used herein, the term "acyl lactylate" is defined as either a pure monolactylate, a pure dilactylate, a pure trilactylate, a mixture of monolactylate and dilactylate or a mixture of monolactylate, dilactylate and trilactylate.

In an embodiment of the invention, component a) is sodium lauroyl lactylate.

In an embodiment of the invention, the amount of acyl lactylate, that is, component a), is from 4 to 12% wt., preferably from 6 to 12% wt., more preferably from 8.5 to 11.5% wt., based on the total weight of the composition.

In another embodiment of the invention, the amount of acyl lactylate, that is, component a), is from 1.3 to 8.8% wt., preferably from 2 to 6.3% wt., more preferably from 2.5 to 6.3% wt., based on the total weight of the composition.

In another embodiment of the invention, component a) is not liquid at room temperature.

In an embodiment of the invention, component a) cannot be directly poured into a reactor at room temperature.

Acyl lactylates are commercially available. Such acyl lactylates are typically prepared by reacting a fatty acid and lactic acid in presence of sodium hydroxide (NaOH), for examples as disclosed in patent application US 2017/0349525.

In an embodiment of the invention, a process for producing alkyl lactylate comprises the step of reacting a fatty acid and lactic acid at a temperature from 100°C to 250°C in the presence of an alkali catalyst.

In a preferred embodiment of the invention, the process for producing sodium lauroyl lactylate comprises the step of reacting lauric acid and lactic acid at a temperature from 100°C to 250°C in the presence of NaOH.

The composition of the invention comprises a mixture of amphoteric surfactants, wherein the mixture comprises
b1) at least an alkyl or alkenyl hydroxysultaine
b2) at least an alkyl or alkenyl amidopropyl hydroxysultaine

### Alkyl or alkenyl hydroxysultaine (bl):

The present invention comprises at least an alkyl or alkenyl hydroxysultaine.

The term "alkyl or alkenyl hydroxysultaine" refers to a compound which consists of an alkyl or alkenyl group covalently bound to a hydroxysultaine moiety, preferably to the nitrogen atom of a hydroxysultaine moiety.

The term "hydroxysultaine" refer to a -N(CH₃)₂-CH₂-CH(OH)-CH₂-SO₃⁻ moiety.

Preferably, the alkyl or alkenyl hydroxysultaine is represented by formula (II) 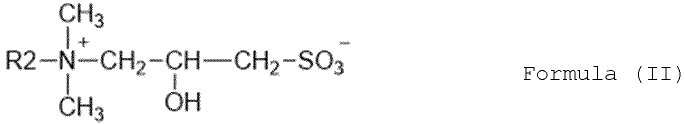 wherein R2 is a linear or branched alkyl or alkenyl group containing from 5 to 23 carbon atoms, preferably from 9 to 17 carbon atoms, more preferably from 9 to 15 carbon atoms, and from 0 to 3 double bonds.

In one embodiment, R2 is a linear or branched alkyl group containing from 5 to 23 carbon atoms, preferably from 9 to 17 carbon atoms, more preferably from 9 to 15 carbon atoms.

In one embodiment, R2 is a linear alkyl group containing from 5 to 23 carbon atoms, preferably from 9 to 17 carbon atoms, more preferably from 9 to 15 carbon atoms.

Examples of hydroxysultaines of formula (II) are lauryl hydroxysultaine, coco hydroxysultaine, cetyl hydroxysultaine, myristyl hydroxysultaine.

In an embodiment of the invention, component b1) is lauryl hydroxysultaine.

In an embodiment of the invention, the amount of alkyl or alkenylhydroxysultaine, i.e. component b1) is from 9 to 24% wt., preferably from 10 to 20% wt., more preferably from 12 to 18% wt., based on the total weight of the composition.

In another embodiment of the invention, the amount of alkyl or alkenylhydroxysultaine, i.e. component b1) is from 1.9 to 13.1% wt., preferably from 3 to 9.4% wt., more preferably from 3.8 to 9.4% wt., based on the total weight of the composition.

Alkyl or alkenylhydroxysultaines are commercially available. They are typically prepared by reacting a tertiary amine, such as a dimethylalkylamine, with sulfonated epichlorhydrin.

### Alkyl or alkenyl amidopropyl hydroxysultaine (b2):

The present invention comprises at least an alkyl or alkenyl amidopropyl hydroxysultaine. The term "alkyl or alkenyl amidopropyl hydroxysultaine" refers to a compound which consists of an alkyl or alkenyl group covalently bound to an amidopropyl moiety, preferably to the carbon atom of the carbonyl group of the amidopropyl moiety, said amidopropyl moiety being in turn covalently bound to a hydroxysultaine moiety, preferably to the nitrogen atom of a hydroxysultaine moiety.

Preferably, the alkyl or alkenyl amidopropyl hydroxysultaine is represented by formula (III) 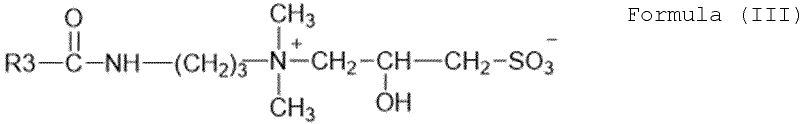 wherein R3 is a linear or branched alkyl or alkenyl group containing from 5 to 23 carbon atoms, preferably from 7 to 21 carbon atoms, more preferably from 7 to 17 carbon atoms, and from 0 to 3 double bonds.

In one embodiment, R3 is a linear or branched alkyl group containing from 5 to 23 carbon atoms, preferably from 7 to 21 carbon atoms, more preferably from 7 to 17 carbon atoms.

In one embodiment, R3 is a linear alkyl group containing from 5 to 23 carbon atoms, preferably from 7 to 21 carbon atoms, more preferably from 7 to 17 carbon atoms.

Examples of alkyl- or alkenylamidopropyl hydroxysultaines of formula (III) are capryl/capramidopropyl hydroxysultaine, cocamidopropyl hydroxysultaine, erucamidopropyl hydroxysultaine, lauramidopropyl hydroxysultaine, myristamidopropyl hydroxysultaine, oleamidopropyl hydroxysultaine, tallowamidopropyl hydroxysultaine.

In an embodiment of the invention, component b2) is cocamidopropyl hydroxysultaine.

In an embodiment of the invention, the amount of component b2) is from 9 to 24% wt., preferably from 10 to 20% wt., more preferably from 12 to 18% wt., based on the total weight of the composition.

In another embodiment of the invention, the amount of component b2) is from 1.9 to 13.1% wt., preferably from 3 to 9.4% wt., more preferably from 3.8 to 9.4% wt., based on the total weight of the composition.

Alkyl or alkenyl amidopropyl hydroxysultaine are commercially available. They are typically prepared by reacting an amidoamine with sulfonated epichlorhydrin.

In another embodiment, the weight ratio between component b1) and component b2 is from 2:1 to 1:2, preferably from 1.5:1 to 1:1.5, more preferably from 1.2:1 to 1:1.2.

In another embodiment, the weight ratio between component a) and component b), that is, the weight ratio between component a) and the sum of component b1) and component b2) is from 1:2.3 to 0.5:9.5, preferably from 1:2 to 1:7, more preferably from 1:2.5 to 1:4.

In one embodiment, in the composition of the invention:
- component a) is a compound of formula (I), wherein R1 is a linear alkyl chain having from 9 to 15 carbon atoms;
- component b1) is a compound of formula (II), wherein R2 is a linear alkyl chain having from 9 to 15 carbon atoms; and
- component b2) is a compound of formula (III), wherein R3 is a linear alkyl chain having from 7 to 17 carbon atoms.

In another embodiment, in the composition of the invention:
- component a) is sodium lauroyl lactylate;
- component b1) is lauryl hydroxysultaine; and
- component b2) is cocamidopropyl hydroxysultaine.

In an embodiment of the invention, the amount of component a), component b1) and component b2) is from 10 to 45%wt., preferably from 25 to 45% wt., preferably from 25 to 43% wt., more preferably from 30 to 43% wt., even more preferably from 35 to 42% wt., based on the total weight of the composition. In a preferred embodiment, the amount of component a), component b1) and component b2) is from 25 to 45% wt., based on the total weight of the composition. In another preferred embodiment, the amount of component a), component b1) and component b2) is from 35 to 42% wt., based on the total weight of the composition.

In an embodiment of the invention, the amount of component a), component b1) and component b2) is from 10 to 45%wt., preferably from 25 to 45% wt., preferably from 25 to 43% wt., more preferably from 30 to 43% wt., even more preferably from 35 to 42% wt., based on the total weight of the composition, wherein the composition further comprises water. In a preferred embodiment, the amount of component a), component b1) and component b2) is from 25 to 45% wt., based on the total weight of the composition, wherein the composition further comprises water. In another preferred embodiment, the amount of component a), component b1) and component b2) is from 35 to 42% wt., based on the total weight of the composition, wherein the composition further comprises water.

In another embodiment, the amount of water in the composition is from 55 to 90% wt., preferably from 55 to 75% wt., preferably from 57 to 75% wt., more preferably from 57 to 70% wt., even more preferably from 58 to 68% wt., based on the total weight of the composition.

In another embodiment, the amount of component a), component b1) and component b2) is from 10 to 45%wt., preferably from 25 to 45% wt., preferably from 25 to 43% wt., more preferably from 30 to 43% wt., even more preferably from 35 to 42% wt., based on the total weight of the composition, the composition further comprises water, and the amount of water in the composition is from 55 to 90% wt., preferably from 55 to 75% wt., preferably from 57 to 75% wt., more preferably from 57 to 70% wt., even more preferably from 58 to 68% wt., based on the total weight of the composition. In a preferred embodiment, the amount of component a), component b1) and component b2) is from 25 to 45% wt., based on the total weight of the composition, the composition further comprises water, and the amount of water in the composition is from 55 to 90% wt., preferably from 55 to 75% wt., preferably from 57 to 75% wt., more preferably from 57 to 70% wt., even more preferably from 58 to 68% wt., based on the total weight of the composition. In another preferred embodiment, the amount of component a), component b1) and component b2) is from 35 to 42% wt., based on the total weight of the composition, the composition further comprises water, and the amount of water in the composition is from 55 to 90% wt., preferably from 55 to 75% wt., preferably from 57 to 75% wt., more preferably from 57 to 70% wt., even more preferably from 58 to 68% wt., based on the total weight of the composition.

In an embodiment of the invention, the viscosity of the composition is from 10000 to 20000 cP. In particular, this viscosity if for compositions wherein the amount of component a), component b1) and component b2) is from 10 to 45%wt., preferably from 25 to 45% wt., preferably from 25 to 43% wt., more preferably from 30 to 43% wt., even more preferably from 35 to 42% wt., based on the total weight of the composition, the composition further comprises water, and wherein the amount of water in the composition is from 55 to 90% wt., preferably from 55 to 75% wt., preferably from 57 to 75% wt., more preferably from 57 to 70% wt., even more preferably from 58 to 68% wt., based on the total weight of the composition. In particular, this viscosity if for compositions wherein the amount of component a), component b1) and component b2) is from 25 to 45% wt., based on the total weight of the composition, the composition further comprises water, and wherein the amount of water in the composition is from 55 to 90% wt., preferably from 55 to 75% wt., preferably from 57 to 75% wt., more preferably from 57 to 70% wt., even more preferably from 58 to 68% wt., based on the total weight of the composition. Alternatively, this viscosity if for compositions wherein the amount of component a), component b1) and component b2) is from 35 to 42% wt., based on the total weight of the composition, the composition further comprises water, and wherein the amount of water in the composition is from 55 to 90% wt., preferably from 55 to 75% wt., preferably from 57 to 75% wt., more preferably from 57 to 70% wt., even more preferably from 58 to 68% wt., based on the total weight of the composition.

In another embodiment, the composition is a clear viscous liquid. In particular, the composition is a clear viscous liquid for compositions wherein the amount of component a), component b1) and component b2) is from 10 to 45%wt., preferably from 25 to 45% wt., preferably from 25 to 43% wt., more preferably from 30 to 43% wt., even more preferably from 35 to 42% wt., based on the total weight of the composition, the composition further comprises water, and the amount of water in the composition is from 55 to 90% wt., preferably from 55 to 75% wt., preferably from 57 to 75% wt., more preferably from 57 to 70% wt., even more preferably from 58 to 68% wt., based on the total weight of the composition. In particular, the composition is a clear viscous liquid for compositions wherein the amount of component a), component b1) and component b2) is from 25 to 45% wt., based on the total weight of the composition, the composition further comprises water, and the amount of water in the composition is from 55 to 90% wt., preferably from 55 to 75% wt., preferably from 57 to 75% wt., more preferably from 57 to 70% wt., even more preferably from 58 to 68% wt., based on the total weight of the composition. Alternatively, in particular the composition is a clear viscous liquid for compositions wherein the amount of component a), component b1) and component b2) is from 35 to 42% wt., based on the total weight of the composition, the composition further comprises water, and the amount of water in the composition is from 55 to 90% wt., preferably from 55 to 75% wt., preferably from 57 to 75% wt., more preferably from 57 to 70% wt., even more preferably from 58 to 68% wt., based on the total weight of the composition.

The term "viscous liquid" refers to a liquid having a viscosity of from 10000 to 20000cP.

In another embodiment of the invention, the amount of component a), component b1) and component b2) is from 5 to 30% wt., preferably from 8 to 30% wt., more preferably from 8 up to (but not including) 25% wt., even more preferably from 10 up to (but not including) 25% wt., based on the total amount of the composition. In a preferred embodiment, the amount of component a), component b1) and component b2) is from 10 up to (but not including) 25% wt., based on the total weight of the composition.

In another embodiment, the amount of component a), component b1) and component b2) is from 5 to 30% wt., preferably from 8 to 30% wt., more preferably from 8 up to (but not including) 25% wt., even more preferably from 10 up to (but not including) 25% wt., based on the total amount of the composition., wherein the composition further comprises water. In a preferred embodiment, the amount of component a), component b1) and component b2) is from 10 up to (but not including) 25% wt., based on the total weight of the composition, wherein the composition further comprises water.

In another embodiment, the amount of water in the diluted composition is from 65 to 95% wt., preferably from 70 to 92% wt., more preferably from 75 to 92% wt., even more preferably from 75 to 90% wt., based on the total weight of the composition.

In another embodiment, the amount of component a), component b1) and component b2) is from 5 to 30% wt., preferably from 8 to 30% wt., more preferably from 8 up to (but not including) 25% wt., even more preferably from 10 up to (but not including) 25% wt., based on the total amount of the composition, the composition further comprises water, and the amount of water in the composition is from 65 to 95% wt., preferably from 70 to 92% wt., more preferably from 75 to 92% wt., even more preferably from 75 to 90% wt., based on the total weight of the composition. In a preferred embodiment, the amount of component a), component b1) and component b2) is from 10 up to (but not including) 25% wt., based on the total weight of the composition, wherein the composition further comprises water, and the amount of water in the composition is from 65 to 95% wt., preferably from 70 to 92% wt., more preferably from 75 to 92% wt., even more preferably from 75 to 90% wt., based on the total weight of the composition.

In an embodiment of the invention, the composition of the invention wherein the amount of component a), component b1) and component b2) is from 5 to 30% wt., preferably from 8 to 30% wt., more preferably from 8 up to (but not including) 25% wt., even more preferably from 10 up to (but not including) 25% wt., based on the total amount of the composition, and wherein the composition further comprises water are suitable for cosmetic applications. In a preferred embodiment of the invention, the composition of the invention wherein the amount of component a), component b1) and component b2) is from 10 up to (but not including) 25% wt., based on the total amount of the composition, and wherein the composition further comprises water, are suitable for cosmetic applications.

In another embodiment, the composition comprises:
- between 5 and 35% wt., preferably between 8 and 30% wt., more preferably between 8 up to (but not including) 25% wt., more preferably from 10 up to (but not including) 25% wt. of acyl lactylate a), alky/alkenyl hydroxysultaine b1) and alkyl/alkenyl amidopropyl hydroxysultaine b2); and
- between 65 and 95% wt., preferably between 70 to 92 %wt., more preferably between 75 to 90 % wt., even more preferably between 75 and 90% wt. of water,
wherein each of the indicated amounts are expressed as percentage by weight of the mentioned component with respect to the total weight of the composition.

In another embodiment of the invention, the composition further comprises a preservative.

Preferably, said preservative is present in compositions of the invention wherein the amount of component a), component b1) and component b2) is from 5 to 30% wt., preferably from 8 to 30% wt., more preferably from 8 up to (but not including) 25% wt., even more preferably from 10 up to (but not including) 25% wt., based on the total amount of the composition, and wherein the composition further comprises water. Preferably, said preservative is present in compositions of the invention wherein the amount of component a), component b1) and component b2) is from 10 up to (but not including) 25% wt., based on the total amount of the composition, and wherein the composition further comprises water.

Examples of suitable preservatives include sorbic acid, sodium benzoate, potassium sorbate, phenoxyethanol, ethylhexylglycerin, caprylyl glycol, decylene glycol, benzoic acid, dehydroacetic acid, propanediol, phenethyl alcohol, undecyl alcohol, tocopherol, methylpropanediol and mixtures thereof.

In an embodiment of the invention, the composition contains a preservative in an amount from 0.1 to 1.5% wt., based on the total weight of the cosmetic composition.

In another embodiment, the composition comprises:
- between 5 and 35% wt., preferably between 8 and 30% wt., more preferably between 8 up to (but not including) 25% wt., more preferably from 10 up to (but not including) 25% wt. of acyl lactylate a), alky/alkenyl hydroxysultaine b1) and alkyl/alkenyl amidopropyl hydroxysultaine b2);
- between 65 and 95% wt., preferably between 70 to 92 %wt., more preferably between 75 to 90 % wt., even more preferably between 75 and 90% wt. of water; and
- between 0.1 to 1.5%wt. of preservative,
wherein each of the indicated amounts are expressed as percentage by weight of the mentioned component with respect to the total weight of the composition.

In another embodiment of the invention, the composition further comprises a perfume.

Preferably, said perfume is present in compositions of the invention wherein the amount of component a), component b1) and component b2) is from 5 to 30% wt., preferably from 8 to 30% wt., more preferably from 8 up to (but not including) 25% wt., even more preferably from 10 up to (but not including) 25% wt., based on the total amount of the composition, and wherein the composition further comprises water. Preferably, said perfume is present in compositions of the invention wherein the amount of component a), component b1) and component b2) is from 10 up to (but not including) 25% wt., based on the total amount of the composition, and wherein the composition further comprises water.

By incorporating perfumes (mixture of fragrant essential oils and aroma compounds, fixatives and solvent used to give the human body, objects, and living spaces a lasting and pleasant smell) in the cosmetic composition of the invention, it is possible to obtain a pleasant smell when using the cosmetic composition for the cleansing of skin and/or hair.

As used herein, the term "perfume" includes a perfume raw material and a perfume combination. Used in this document, the term "combination" refers to a mixture of two or more perfume raw material.

Suitable perfume oils are mixtures of natural and/or synthetic ingredients. A non-exhaustive list of natural ingredients include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamon, costus, iris, calamus), woods (pinewood, sandalwood, guaiac wood, cedarwood, rosewood), herbs and grasses (tarragon, lemon grass, sage, thyme, needles and branches (spruce, fir, pine, dwarf pine), resins and balsams (galbanum, elemi, benzoin, myrrh, olibanum, opoponax).

Typical synthetic fragances include, among others, alcohols, ketones, aldehydes, esters, ethers, nitrites, and alkenes such as terpenes.

In an embodiment of the invention, the composition contains perfume in an amount from 0.1 to 4.0% wt., preferably from 0.1 to 3.0% wt., based on the total weight of the cosmetic composition.

In another embodiment, the composition comprises:
- between 5 and 35% wt., preferably between 8 and 30% wt., more preferably between 8 up to (but not including) 25% wt., more preferably from 10 up to (but not including) 25% wt. of acyl lactylate a), alky/alkenyl hydroxysultaine b1) and alkyl/alkenyl amidopropyl hydroxysultaine b2);
- between 65 and 95% wt., preferably between 70 to 92 %wt., more preferably between 75 to 90 % wt., even more preferably between 75 and 90% wt. of water; and
- between 0.1 and 4.0% wt., preferably between 0.1 and 3.0%, of perfume,
wherein each of the indicated amounts are expressed as percentage by weight of the mentioned component with respect to the total weight of the composition.

In another embodiment, the composition comprises:
- between 5 and 35% wt., preferably between 8 and 30% wt., more preferably between 8 up to (but not including) 25% wt., more preferably from 10 up to (but not including) 25% wt. of acyl lactylate a), alky/alkenyl hydroxysultaine b1) and alkyl/alkenyl amidopropyl hydroxysultaine b2);
- between 65 and 95% wt., preferably between 70 to 92 %wt., more preferably between 75 to 90 % wt., even more preferably between 75 and 90% wt. of water;
- between 0.1 to 1.5% wt. of preservative, and
- between 0.1 and 4.0%, preferably between 0.1 and 3.0% of perfume,
wherein each of the indicated amounts are expressed as percentage by weight of the mentioned component with respect to the total weight of the composition.

The pH of the composition of the present invention is comprised between 5.0 and 7.5, preferably between 5.0 and 7.0, more preferably from 5.5 and 7.0, even more preferably from 5.5 to 6.5.

Preferably, said pH is for compositions of the invention wherein the amount of component a), component b1) and component b2) is from 5 to 30% wt., preferably from 8 to 30% wt., more preferably from 8 up to (but not including) 25% wt., even more preferably from 10 up to (but not including) 25% wt., based on the total amount of the composition, and wherein the composition further comprises water. Preferably, said pH is for compositions of the invention wherein the amount of component a), component b1) and component b2) is from 10 up to (but not including) 25% wt., based on the total amount of the composition, and wherein the composition further comprises water.

The viscosity of the composition is from 2000 to 20000 cP.

Preferably, said viscosity is for compositions of the invention wherein the amount of component a), component b1) and component b2) is from 5 to 30% wt., preferably from 8 to 30% wt., more preferably from 8 up to (but not including) 25% wt., even more preferably from 10 up to (but not including) 25% wt., based on the total amount of the composition, and wherein the composition further comprises water. Preferably, said viscosity is for compositions of the invention wherein the amount of component a), component b1) and component b2) is from 10 up to (but not including) 25% wt., based on the total amount of the composition, and wherein the composition further comprises water.

The expression "up to (but not including)" as used herein is equivalent to "up to" when defining the upper limit of a range means that the range defined ends at the upper limit provided but does not include the value of said upper limit.

The sum of the weight percentages of the ingredients of the compositions of the invention cannot exceed 100% wt. That means, that if two or more components are defined as being present in a certain range of weight percentages, the weight percentage of each component shall be selected so that the total amount of ingredients does not exceed 100% wt.

The viscosity of the composition of the invention is a viscosity directly suitable for the cosmetic intended use. In an embodiment of the invention, the composition is free of a thickener.

The expression "free of" as used herein means that the corresponding component is totally absent (i.e. 0 wt%) or substantially absent (i.e. less than 0.1 wt%), i.e. the composition contains 0% to 0.1% by weight of the corresponding component.

The term "thickener" as used herein refers to substances specifically added to formulations to increase their viscosity and provide a desirable texture. Examples of thickeners are natural thickeners, such as xanthan gum, guar gum, agar-agar, sodium alginate, pectin; synthetic thickeners such as carbomers, polyacrylamide, acrylates copolymer; cellulosic thickeners such as hydroxy ethylcellulose, methylcellulose, carboxymethylcellulose; waxes such as beeswax, silica and mineral-based thickener such as silica, kaolin clay, proteins such as hydrolyzed wheat protein, starch-based thickener such as cornstarch.

The composition according to the present invention may also comprise other ingredients suitable for a cosmetic composition, such as oil components, silicone compounds, powders, non-ionic surfactants, anionic surfactants, polymers, metal ion sequestering agents, UV protection factors, vitamins, antioxidants, antioxidant aids, perfume oils, germ inhibitors and the like as further auxiliaries and additives. Preferably, said other ingredients are present in compositions of the invention wherein the amount of component a), component b1) and component b2) is from 5 to 30% wt., preferably from 8 to 30% wt., more preferably from 8 up to (but not including) 25% wt., even more preferably from 10 up to (but not including) 25% wt., based on the total amount of the composition, and wherein the composition further comprises water. Preferably, said other ingredients are present in compositions of the invention wherein the amount of component a), component b1) and component b2) is from 10 up to (but not including) 25% wt., based on the total amount of the composition, and wherein the composition further comprises water.

Examples of oils include liquid oils, solid oils, hydrocarbon oils and synthetic ester oils. Suitable oil components are, for example, Guerbet alcohols based on fatty alcohols containing 6 to 22 and preferably 8 to 10 carbon atoms, esters of linear C6-C22 fatty acids with linear C6-C22 fatty alcohols, esters of branched C6-C22 carboxylic acids with linear C6-C22 fatty alcohols such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isopropyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl cleats, erucyl behenate and erucyl erucate. Also suitable are esters of linear C6-C22 fatty acids with branched alcohols, more particularly 2-ethyl hexanol, esters of hydroxycarboxylic acids with linear or branched C6-C22 fatty alcohols, esters of linear and/or branched fatty acids with polyhydric alcohols for example propylene glycol, dimer diol or trimer triol) and/or Guerbet alcohols, triglycerides based on C6-C10 fatty acids, liquid mono-/di-/triglyceride mixtures based on C6-C18 fatty acids, esters of C6-C22 fatty alcohols and/or Guerbet alcohols with aromatic carboxylic acids, more particularly benzoic acid, esters of C6-C12 dicarboxylic acids with linear or branched alcohols containing 1 to 22 carbon atoms or polyols containing 2 to 10 carbon atoms and 2 to 6 hydroxyl groups, vegetable oils such as avocado oil, almond oil, hazelnut oil, babassu palm oil, borage oil, peanut oil, jojoba oil, canola oil, hemp oil, soybean oil, milk thistle oil, safflower oil, chufa oil, coconut oil, rapeseed oil, black cumin oil, wheat germ oil, sunflower oil, linseed oil, macadamia nut oil, corn oil, walnut oil, olive oil, branched primary alcohols, substituted cyclohexanes, linear and branched C6-C22 fatty alcohol carbonates, Guerbet carbonates, esters of benzoic acid with linear and/or branched C6-C22 alcohols, linear or branched, symmetrical or non-symmetrical dialkyl ethers containing 6 to 22 carbon atoms per alkyl group, ring opening products of epoxidized fatty acid esters with polyols, silicone oils and/or aliphatic or naphthenic hydrocarbons, for example dialkyl cyclohexanes.

Examples of hydrocarbon oils include liquid paraffin, squalane, pristane, paraffin, ceresin, squalene, petrolatum, and microcrystalline wax.

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Preferred silicone compounds are hydrophobic silicone oils, which are silicone oils which are soluble in paraffinic oil at 25°C. Hydrophobic silicone oils to be used according to the present invention include both volatile and non-volatile silicone oils.

Specific examples include a cyclic methyl siloxane having the formula {(CH₃)₂SiO}ₓ in which x is 3-6, or short chain linear methyl siloxanes having the formula ((CH₃)₂SiO{(CH₃)₂SiO}_{y}Si(CH₃)₃ in which y is 0-5.

Some suitable cyclic methyl siloxanes are hexamethylcyclotrisiloxanes (D3), a solid with a boiling point of 134°C and the formula {(Me₂)SiO}₃; octamethylcyclotetrasiloxane (D4) with a boiling point of 176°C, a viscosity of 2.3 mm²/s, and the formula {(Me₂)SiO}₄; decamethylcyclopentasiloxane (D5) (cyclomethicone) with a boiling point of 210°C, a viscosity of 3.87 mm²/s, and the formula {(Me₂)SiO}₅; and dodecamethylcyclohexasiloxane (DE) with a boiling point of 245°C, a viscosity of 6.62 mm²/s and the formula {(Me₂)SiO}₆.

Some suitable short linear methyl siloxane are hexamethyldisiloxane (MM) with a boiling point of 100°C, viscosity of 0-65 mm<2>/s, and formula Me₃SiOMe₃; octamethyltrisiloxane (MDM) with a boiling point of 152°C., viscosity of 1.04 mm²/s, and formula Me₃SiOMe₂SiOSiMe₃; decamethyltetrasiloxane (MD2M) with a boiling point of 194°C, viscosity of 1.53 mm²/s, and formula Me₃SiO(MeSiO)₂SiMe₃; dodecamethylpentasiloxane (MD3M) with a boiling point of 229°C, viscosity of 2.06 mm²/s, and formula Me₃SiO(Me₂SiO)₃SiMe₃; tetradecamethylhexasiloxane (MD4M) with a boiling point of 245°C, viscosity of 2.63 mm²/s, and formula Me₃SiO(Me₂SiO)₄SiMe₃; and hexadecamethylheptasiloxane (MD5M) with a boiling point of 270°C, viscosity of 3.24 mm²/s, and formula Me₃SiO(Me₂SiO)₅SiMe₃.

Furthermore, long chain linear siloxanes such as phenyltrimethicone, bis(phenylpropyl)dimethicone, dimethicone, and dimethiconol are also included.

Examples of powders include inorganic powders such as talc, kaolin, mica, sericite, muscovite, phlogopite, synthetic mica, lepidolite, biotite, vermiculite, bentonite, hectorite, laponite, magnesium carbonate, calcium carbonate, aluminum silicate, barium silicate, calcium silicate, magnesium silicate, strontium silicate, tungstate, magnesium, silica, zeolite, barium sulfate, calcined calcium sulfate (calcined gypsum), calcium phosphate, fluorine apatite, hydroxyapatite, ceramic powder, metallic soap (e.g., zinc myristate, calcium palimitate, and aluminum stearate), and boron nitride; organic powders such as polyamide resin powder (nylon powder), polyethylene powder, polymethylmethacrylate powder, polystyrene powder, styrene-acrylic acid copolymer resin powder, benzoguanamine resin powder, poly(tetrafluroethylene) powder; inorganic white pigments such as titanium dioxide and zinc oxide; inorganic red pigments such as iron oxide (red iron oxide) and iron titanate; inorganic brown pigments such as γ-iron oxide; inorganic yellow pigments such as yellow iron oxide and ocher; inorganic black pigments such as black iron oxide and lower order titanium oxide; inorganic purple pigments such as mango violet and cobalt violet; inorganic green pigments such as chrome oxide, chrome hydroxide, and cobalt titanate; inorganic blue pigments such as ultramarine and Prussian blue; pearl pigments such as titanium oxide coated mica, titanium oxide coated bismuth oxychloride, titanium oxide coated talc, colored titanium oxide coated mica, bismuth oxychloride, and fish scale flakes; metal powder pigments such as aluminum powder and copper powder; organic pigments such as zirconium, barium, or aluminum lake (e.g., organic pigments such as Red No.201, Red No.202, Red No.204, Red No.205, Red No.220, Red No.226, Red No.228, Red No.405, Orange No.203, Orange No.204, Yellow No.205, Yellow No.401, and Blue No.404,or Red No.3, Red No.104, Red No.106, Red No.227, Red No.230, Red No.401, Red No.505, Orange No.205, Yellow No.4, Yellow No.5, Yellow No.202, Yellow No.203, Green No.3, and Blue No.1); and natural colors such as chlorophyll and β-carotene.

Examples of lipophilic nonionic surfactants include sorbitan fatty acid esters (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate); glycerol or polyglycerol fatty acid esters (such as glycerol mono-cotton seed oil fatty acid ester, glycerol monoerucate, glycerol sesquioleate, glycerol monostearate, glycerol-α, α'-oleate pyroglutamate, and glycerol monostearate malate); hardened castor oil derivatives; and glycerol alkyl ethers.

Examples of hydrophilic nonionic surfactants include alkanolamides (such as coconut oil fatty acid diethanolamide, lauric acid monoethanolamide, and fatty acid isopropanolamide); sucrose fatty acid esters; and trioleyl phosphate.

Examples of UV protection factors include organic substances (light filters) which are liquid or crystalline at room temperature and which are capable of absorbing ultraviolet radiation and of releasing the energy absorbed in the form of longer-wave radiation, for example heat. UV-B filters can be oil-soluble or water-soluble. The following are examples of oil-soluble substances: 3-benzylidene camphor or 3-benzylidene norcamphor and derivatives thereof, for example 3-(4-methylbenzylidene)-camphor; 4-aminobenzoic acid derivatives, preferably 4-(dimethylamino)-benzoic acid-2-ethylhexyl ester, 4-(dimethylamino)-benzoic acid-2-octyl ester and 4-(dimethylamino)-benzoic acid Amylester; esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene); esters of salicylic acid, preferably salicylic acid-2-ethylhexyl ester, salicylic acid-4-isopropylbenzyl ester, salicylic acid homomethyl ester; derivatives of benzophenone, preferably 2-hydroxy-4-methoxybenzo-phenone, 2-hydroxy-4-methoxy-4'-methylbenzophenone, 2,2'-dihydroxy-4-methoxybenzophenone; esters of benzalmalonic acid, preferably 4-methoxybenzalmalonic acid di-2-ethylhexyl ester; triazine derivatives such as, for example, 2,4,6-trianilino-(p-carbo-2'-ethyl-1'-hexyloxy)-1,3,5-triazine and Octyl Triazone; propane-1,3-diones such as, for example, 1-(4-tert.butylphenyl)-3-(4T-methoxyphenyl)-propane-1,3-dione; 2-phenylbenzimidazole-5-sulfonic acid and alkali metal, alkaline earth metal, ammonium, alkylammonium, alkanolammonium and glucammonium salts thereof; sulfonic acid derivatives of benzophenones, preferably 2-hydroxy-4-methoxybenzophenone-5-sulfonic acid and salts thereof; sulfonic acid derivatives of 3-benzylidene camphor such as, for example, 4-(2-oxo-3-bornylidenemethyl)-benzene sulfonic acid and 2-methyl-5-(2-oxo-3-bornylidene)-sulfonic acid and salts thereof.

Typical UV-A filters are, in particular, derivatives of benzoyl methane such as, for example 1-(4'-tert.butylphenyl)-3-(4'-methoxyphenyl)-propane-1,3-dione, 4-tert-butyl-4'-methoxydibenzoyl methane (Parsol 1789) or 1-phenyl-3-(4'-isopropylphenyl)-propane-1,3-dione.

The UV-A and UV-B filters may of course also be used in the form of mixtures. Besides the soluble substances mentioned, insoluble pigments, i.e. finely dispersed metal oxides or salts, may also be used for this purpose. Examples of suitable metal oxides are, in particular, zinc oxide and titanium dioxide and also oxides of iron, zirconium, silicon, manganese, aluminium and cerium and mixtures thereof. Silicates (talcum), barium sulfate and zinc stearate may be used as salts. The oxides and salts are used in the form of the pigments for skin-care and skin-protecting emulsions and decorative cosmetics. The particles should have an average diameter of less than 100 nm, preferably from 5 to 50 nm and more preferably from 15 to 30 nm. They may be spherical in shape although ellipsoidal particles or other non-spherical particles may also be used. The pigments may also be surface-treated, i.e. hydrophilicized or hydrophobicized. Typical examples are coated titanium dioxides such as, for example, Titandioxid T 805 (Degussa) or Eusolex T2000 (Merck). Suitable hydrophobic coating materials are, above all, silicones and especially trialkoxyoctyl silanes or simethicones. So-called micro- or nanopigments are preferably used in sun protection products. Micronized zinc oxide is preferably used.

Besides the two above-mentioned groups of primary protection factors, secondary protection factors of the antioxidant type may also be used. Secondary sun protection factors of the antioxidant type interrupt the photochemical reaction chain which is initiated when UV rays penetrate into the skin. Typical examples of suitable antioxidants are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example α-carotene, β-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, γ-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages (also (metal) chelators (for example (α-hydroxyfatty acids, palmitic acid, phytic acid, lactoferrine), α-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example γ-linolenic acid, linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, (α-glycosyl rutin, ferulic acid, furfurylidene glucitol, carnosine, butyl hydroxytoluene, butyl hydroxyanisole, nordihydroguaiac resin acid, nordihydroguaiaretic acid, trihydroxy-butyrophenone, uric acid and derivatives thereof, mannose and derivatives thereof, Superoxid-Dismutase, zinc and derivatives thereof (for example ZnO, ZnSO4), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Examples of metal ion sequestering agents include 1-hydroxyethane-1,1-diphosphonic acid, 1-hydroxyethane-1,1-diphosphonic acid 4Na salt, disodium edetate, trisodium edetate, tetrasorium edetate, sodium citrate, sodium polyphosphate, sodium metaphosphate, gluconic acid, phosphoric acid, citric acid, ascorbic acid, succinic acid, edetic acid, and trisodium hydroxyethyl ethylenediamine triacetate.

Examples of vitamins include vitamins A, B1, B2, B6, C, and E and the derivatives thereof; pantothenic acid and the derivatives thereof; and biotin.

Examples of antioxidants include tocopherols, dibutylhydroxytoluene, butylhydroxyanisole, and gallic acid esters. Examples of antioxidant aids include phosphoric acid, citric acid, ascorbic acid, maleic acid, malonic acid, succinic acid, lactic acid, fumaric acid, cephalin, hexametaphosphates, phytic acid, and ethylenediaminetetraacetic acid.

Typical examples of germ inhibitors are preservatives which act specifically against gram-positive bacteria such as, for example, 2,4,4'-trichloro-2'-hydroxydiphenyl ether, chlorhexidine (1,6-di-(4-chlorophenyl-biguanido)-hexane) or TCC (3,4,4'-trichlorocarbanilide). Numerous perfumes and essential oils also have antimicrobial properties. Typical examples are the active substances eugenol, menthol and thymol in clove, mint and thyme oil.

In an embodiment of the invention, the composition is free of a sulfated anionic surfactant. Sulfated anionic surfactants refers to sodium lauryl sulfate (SLS) or sodium laureth sulfate (SLES). In another embodiment, the composition is free of sodium laureth sulfate and sodium lauryl sulfate.

In another embodiment of the invention, the composition is free of surfactants comprising ethylene oxide (EO) units.

In an embodiment of the invention, a composition free of surfactant comprising EO units refers to a composition free of sodium laureth sulfate (SLES), ammonium laureth sulfate, disodium laureth sulfosuccinate, laureth carboxylic acid, sodium/potassium laureth carboxylate.

### Process to prepare the composition:

The process for producing the composition of the invention comprises the steps of:
i) subjecting component a) to a temperature from 50°C to 80°C, preferably at a temperature from 55°C to 65°C; optionally in the presence of water, so that component a) is in liquid form,
ii) mixing component a) and component b1) and b2), and optionally water, at a temperature from 40°C to 60°C; an
iii) stirring to obtain a homogeneous solution,
wherein there is water in present in step i), step ii) or both steps.

In another embodiment of the invention, the process for producing the composition of the invention comprises the steps of:
i) subjecting component a) to a temperature from 50°C to 80°C, preferably at a temperature from 55°C to 65°C; optionally in the presence of water, so that component a) is in liquid form,
ii) mixing component a) and component b1) and b2), and optionally water, at a temperature from 40°C to 60°C; and
iii) stirring to obtain a homogeneous solution,

wherein there is water in step i, ii or both steps,
wherein the amount of component a), component b1) and component b2) is from 10 to 45%wt., preferably from 25 to 45% wt., preferably from 25 to 43% wt., more preferably from 30 to 43% wt., even more preferably from 35 to 42% wt., based on the total amount of the composition, wherein the composition further comprises water. In a particular embodiment of said process, the amount of component a), component b1) and component b2) is from 25 to 45%wt., based on the total amount of the composition, wherein the composition further comprises water. In another particular embodiment of said process, the amount of component a), component b1) and component b2) is from 35 to 42%wt., based on the total amount of the composition, wherein the composition further comprises water.

In another embodiment of the invention, the process for producing the composition of the invention comprises a step before step i) comprising the preparation of component a) by reaction of lactic acid with a fatty acid, preferably a fatty acid of formula R1-COOH, wherein R1 is as previously defined, at a temperature from 100°C to 250°C in the presence of an alkali catalyst, such as sodium hydroxide; and the cooling down of the obtained product to a temperature from 50°C to 80°C. This cooling down step would already correspond to step i) of previously defined process of subjecting component a) to a temperature from 50°C to 80°C.

Thus, in a particular embodiment, the process for producing the composition of the invention comprises the steps of:
0) preparing component a) by reaction of lactic acid with a fatty acid, preferably a fatty acid of formula R1-COOH, wherein R1 is as previously defined, at a temperature from 100°C to 250°C in the presence of an alkali catalyst, such as sodium hydroxide;
i) cooling down of the product obtained in step 0) to a temperature from 50°C to 80°C, preferably at a temperature from 55°C to 65°C; optionally in the presence of water, so that component a) is in liquid form,
ii) mixing component a) and component b1) and b2), and optionally water, at a temperature from 40°C to 60°C; and
iii) stirring to obtain a homogeneous solution,
wherein there is water in present in step i), step ii) or both steps.

Preferably, the above process comprising step 0) is carried out for compositions of the invention wherein the amount of component a), component b1) and component b2) is from 10 to 45%wt., preferably from 25 to 45% wt., preferably from 25 to 43% wt., more preferably from 30 to 43% wt., even more preferably from 35 to 42% wt., based on the total amount of the composition, wherein the composition further comprises water. Preferably, the above process comprising step 0) is carried out for compositions of the invention wherein the amount of component a), component b1) and component b2) is from 25 to 45% wt., based on the total amount of the composition, wherein the composition further comprises water. Preferably, the above process comprising step 0) is carried out for compositions of the invention wherein the amount of component a), component b1) and component b2) is from 35 to 42% wt., based on the total amount of the composition, wherein the composition further comprises water.

By using the residual heat of the preparation of component a), it is possible to obtain a more energy-efficient process, thus leading to a reduction in CO₂ emissions.

In another embodiment of the invention, the process for producing the composition of the invention wherein the amount of component a), component b1) and component b2) in the composition is from 5 to 35% wt., preferably from 8 to 30% wt., more preferably from 8 up to (but not including) 25% wt., even more preferably from 10 up to (but not including) 25% wt. based on the total amount of the composition, and the process comprises the steps of:
1. mixing the a composition of the invention wherein the amount of component a), component b1) and component b2) is from 10 to 45%wt., preferably from 25 to 45% wt., preferably from 25 to 43% wt., more preferably from 30 to 43% wt., even more preferably from 35 to 42% wt., based on the total amount of the composition, wherein the composition further comprises water, with water at a temperature suitable for homogenization thereof, and
2. stirring to obtain a homogeneous solution,
wherein the total concentration of components a), b1) and b2) in the composition mixed with water in step 1) is higher than that of the composition obtained after mixing with water. In a particular embodiment of said process, in the composition that is produced the amount of component a), component b1) and component b2) in the composition is from 10 up to (but not including) 25% wt., based on the total amount of the composition, and the composition of step a) that is mixed with water has an amount of component a), component b1) and component b2) of from 25 to 45%wt., based on the total amount of the composition, wherein the composition further comprises water. In another particular embodiment of said process, in the composition that is produced the amount of component a), component b1) and component b2) in the composition is from 10 up to (but not including) 25% wt., based on the total amount of the composition, and the composition of step a) that is mixed with water has an amount of component a), component b1) and component b2) of from 35 to 42%wt., based on the total amount of the composition, wherein the composition further comprises water.

In an embodiment, the temperature suitable for homogenization in step i) is room temperature.

In another embodiment of the invention, the process for producing the composition of the invention wherein the amount of component a), component b1) and component b2) in the composition is from 5 to 35% wt., preferably from 8 to 30% wt., more preferably from 8 up to (but not including) 25% wt., even more preferably from 10 up to (but not including) 25% wt. based on the total amount of the composition, and the process comprises the steps of:
1. mixing a composition comprising components a), b1) and b2) with water, wherein said composition is obtained by subjecting component a) to a temperature from 50°C to 80°C, preferably at a temperature from 55°C to 65°C; optionally in the presence of water, so that component a) is in liquid form; mixing component a) and component b1) and b2) at a temperature from 40°C to 60°C; and optionally water; stirring to obtain a homogeneous solution, wherein there is water in step i, ii or both steps, wherein the amount of component a), component b1) and component b2) is from 10 to 45%wt., preferably from 25 to 45% wt., preferably from 25 to 43% wt., more preferably from 30 to 43% wt., even more preferably from 35 to 42% wt., based on the total amount of the composition, and
2. stirring to obtain a homogeneous solution,
wherein the total concentration of components a), b1) and b2) in the composition mixed with water in step 1) is higher than that of the composition obtained after mixing with water. In a particular embodiment of said process, in the composition that is produced the amount of component a), component b1) and component b2) in the composition is from 10 up to (but not including) 25% wt., based on the total amount of the composition, and the composition of step a) that is mixed with water has an amount of component a), component b1) and component b2) of from 25 to 45%wt., based on the total amount of the composition, wherein the composition further comprises water. In another particular embodiment of said process, in the composition that is produced the amount of component a), component b1) and component b2) in the composition is from 10 up to (but not including) 25% wt., based on the total amount of the composition, and the composition of step a) that is mixed with water has an amount of component a), component b1) and component b2) of from 35 to 42%wt., based on the total amount of the composition, wherein the composition further comprises water.

In another embodiment of the invention, the process comprising steps 1 and 2 defined above further comprises the step of adding a perfume. Said step is preferably carried out before or after step 2.

In another embodiment of the invention, the process comprising steps 1 and 2 defined above further comprises the step of adding a preservative. Said step is preferably carried out before or after step 2.

In another embodiment of the invention, the process comprising steps 1 and 2 defined above further comprises the step of adding a perfume and a preservative. Said step is preferably carried out before or after step 2.

For the preparation of the composition wherein the amount of component a), component b1) and component b2) is from 5 to 35% wt., preferably from 8 to 30% wt., more preferably from 8 up to (but not including) 25% wt., even more preferably from 10 up to (but not including) 25% wt. based on the total amount of the composition (in particular wherein the amount of component a), component b1) and component b2) is from 10 up to (but not including) 25% wt., based on the total weight of the composition)it is not needed to previously heat the solution comprising acyl lactylate a), alky/alkenyl hydroxysultaine b1) and alkyl/alkenyl amidopropyl hydroxysultaine b2) prior to the mixing with water, thus resulting in an enhancement of ingredients stability, as the mixing at room temperature helps preserve the integrity and efficacy of sensitive compounds, which can degrade or lose potency when exposed to high temperatures; and in an increase in energy efficiency, as the amount of energy used to prepare the cosmetic composition is lower, resulting in lower operational costs and in a significant reduction of greenhouse gas emissions.

The compositions of the invention, preferably those wherein the amount of component a), component b1) and component b2) is from 5 to 35% wt., preferably from 8 to 30% wt., more preferably from 8 up to (but not including) 25% wt., even more preferably from 10 up to (but not including) 25% wt. based on the total amount of the composition (in particular wherein the amount of component a), component b1) and component b2) is from 10 up to (but not including) 25% wt., based on the total weight of the composition), can be used in a large number of types of product for the skin and/or the hair such as mousses, gels, masks for the face or the hair, shampoo, conditioners, formulations for improving hairstyling, or for facilitating the combing or disentangling of the hair, for providing volume or sheen, rinsing formulations, compositions for dying or colouring the hair, hand and body lotions and oils, products for improving the moisturization of the skin, cleansing milks, make-up-removing compositions, creams or lotions for protecting against the sun and ultraviolet radiation, care and/or treatment milks and creams, mascaras, products intended to be applied to the lips or other mucous membranes, sticks, deodorant and antiperspirant products, shaving lotions, bath oils, talcs and other compositions of the same type, alternatively, the composition can also be used in cleansing products for companion animals.

In an embodiment of the invention, the compositions of the invention, preferably those wherein the amount of component a), component b1) and component b2) is from 5 to 35% wt., preferably from 8 to 30% wt., more preferably from 8 up to (but not including) 25% wt., even more preferably from 10 up to (but not including) 25% wt. based on the total amount of the composition (in particular wherein the amount of component a), component b1) and component b2) is from 10 up to (but not including) 25% wt., based on the total weight of the composition), can be used as cosmetic compositions. In another embodiment, the compositions of the invention, preferably those wherein the amount of component a), component b1) and component b2) is from 5 to 35% wt., preferably from 8 to 30% wt., more preferably from 8 up to (but not including) 25% wt., even more preferably from 10 up to (but not including) 25% wt. based on the total amount of the composition (in particular wherein the amount of component a), component b1) and component b2) is from 10 up to (but not including) 25% wt., based on the total weight of the composition), are used for hair care and/or for skin care. In another embodiment, the compositions of the invention, preferably those wherein the amount of component a), component b1) and component b2) is from 5 to 35% wt., preferably from 8 to 30% wt., more preferably from 8 up to (but not including) 25% wt., even more preferably from 10 up to (but not including) 25% wt. based on the total amount of the composition (in particular wherein the amount of component a), component b1) and component b2) is from 10 up to (but not including) 25% wt., based on the total weight of the composition), can be used in compositions for the cleansing of skin and/or hair, hair dyeing compositions, and compositions for conditioning and moisturizing of skin and/or hair, in another embodiment, the compositions of the invention, preferably those wherein the amount of component a), component b1) and component b2) is from 5 to 35% wt., preferably from 8 to 30% wt., more preferably from 8 up to (but not including) 25% wt., even more preferably from 10 up to (but not including) 25% wt. based on the total amount of the composition (in particular wherein the amount of component a), component b1) and component b2) is from 10 up to (but not including) 25% wt., based on the total weight of the composition), can be used in compositions for companion animal cleansing, such as pet shampoos and pet skin cleansers.

Thus, a further aspect refers to the use of the compositions of the invention, preferably those wherein the amount of component a), component b1) and component b2) is from 5 to 35% wt., preferably from 8 to 30% wt., more preferably from 8 up to (but not including) 25% wt., even more preferably from 10 up to (but not including) 25% wt. based on the total amount of the composition (in particular wherein the amount of component a), component b1) and component b2) is from 10 up to (but not including) 25% wt., based on the total weight of the composition), for the care of skin and/or hair selected from the group consisting of cleansing of skin and/or hair, conditioning and moisturizing of skin and/or hair, and the dyeing of hair, preferably cleansing of skin and/or hair.

A further aspect refers to the use of the compositions of the invention, preferably those wherein the amount of component a), component b1) and component b2) is from 5 to 35% wt., preferably from 8 to 30% wt., more preferably from 8 up to (but not including) 25% wt., even more preferably from 10 up to (but not including) 25% wt. based on the total amount of the composition (in particular wherein the amount of component a), component b1) and component b2) is from 10 up to (but not including) 25% wt., based on the total weight of the composition), to prepare a cosmetic composition for the care of skin and/or hair selected from the group consisting of cleansing of skin and/or hair, conditioning and moisturizing of skin and/or hair, and the dyeing of hair, preferably cleansing of skin and/or hair.

In a preferred embodiment, the compositions of the invention, preferably those wherein the amount of component a), component b1) and component b2) is from 5 to 35% wt., preferably from 8 to 30% wt., more preferably from 8 up to (but not including) 25% wt., even more preferably from 10 up to (but not including) 25% wt. based on the total amount of the composition (in particular wherein the amount of component a), component b1) and component b2) is from 10 up to (but not including) 25% wt., based on the total weight of the composition), are used for the cleansing of skin and/or hair. In another embodiment, the compositions of the invention, preferably those wherein the amount of component a), component b1) and component b2) is from 5 to 35% wt., preferably from 8 to 30% wt., more preferably from 8 up to (but not including) 25% wt., even more preferably from 10 up to (but not including) 25% wt. based on the total amount of the composition (in particular wherein the amount of component a), component b1) and component b2) is from 10 up to (but not including) 25% wt., based on the total weight of the composition), are used for the cleansing of skin and/or hair of companion animals.

A method of cleansing skin and/or hair is also part of the invention, wherein the method comprises the steps of wetting or dampening the skin and/or hair, applying the composition of the invention, preferably wherein the amount of component a), component b1) and component b2) is from 5 to 35% wt., preferably from 8 to 30% wt., more preferably from 8 up to (but not including) 25% wt., even more preferably from 10 up to (but not including) 25% wt. based on the total amount of the composition (in particular wherein the amount of component a), component b1) and component b2) is from 10 up to (but not including) 25% wt., based on the total weight of the composition), on the skin and/or hair, and rinsing the skin and/or hair with water.

In particular, in the methods defined above a sufficient amount of the composition of the invention, preferably wherein the amount of component a), component b1) and component b2) is from 5 to 35% wt., preferably from 8 to 30% wt., more preferably from 8 up to (but not including) 25% wt., even more preferably from 10 up to (but not including) 25% wt. based on the total amount of the composition (in particular wherein the amount of component a), component b1) and component b2) is from 10 up to (but not including) 25% wt., based on the total weight of the composition), is applied. The term "sufficient amount" refers to the amount of cosmetic composition necessary to achieve the cleansing or conditions of skin and/or hair and can be easily determined by the skilled person. An example of a sufficient amount is from 0.2 g to 20 g, more preferably from 0.5 g to 15 g.

As used herein, viscosity is measured in a Brookfield LVT viscometer at 20°C with a spindle 2 at 30 rpm.

As used herein, "room temperature" is understood as a temperature from 15 to 30°C, preferably from 20 to 30°C, more preferably from 20 to 25°C.

As used herein, a "clear" composition is understood as a transparent composition. As used herein, a "clear" or "transparent" appearance of a mixture/composition/formulation refers to the optical transmittance being of at least 70%, preferably at least 80%. Preferably, and unless specified otherwise, the optical transmittance of such mixture/composition/formulation is measured at λ = 600 nm (1 cm thickness, 20 °C).

As used herein, a stable composition refers to a composition which maintains its appearance, color, or viscosity, or with little variation, within an interval of time, immediately after their preparation and after storage.

The following examples are given in order to provide a person skilled in the art with a sufficiently clear and complete explanation of the present invention but should not be considered as limiting of the essential aspects of its subject, as set out in the preceding portions of this description.

### EXAMPLES:

The first part of the Examples section corresponds to the preparation of the compositions according to the invention. The second part of the Examples section refers to the performance of the composition of the present invention.

### Example 1

### Preparation of Composition A (comparative A)

For the formulation of Composition A, the corresponding amount of Sodium Lauroyl Lactylate was filled into a vessel and melted at 60°C. Afterwards, Lauryl Hydroxysultaine was filled into the vessel charge. The mixture was stirred at 60°C until complete homogenization of the surfactants and afterwards sodium citrate was added into the mixture and stirred until complete homogenization. Afterwards, the corresponding amount of water was added, and the pH of the mixture was adjusted to pH=5.5-6.5 with sodium hydroxide.

### Preparation of Composition B (comparative B)

For the formulation of Composition B, the corresponding amount of Sodium Lauroyl Lactylate was filled into a vessel and melted at 60°C. Afterwards, Cocamidopropyl Hydroxysultaine was filled into a vessel charge. The mixture was stirred at 60°C until complete homogenization of the surfactants and afterwards sodium citrate was added into the mixture and stirred until complete homogenization. Afterwards, the corresponding amount of water was added, and the pH of the mixture was adjusted to pH=5.5 - 6.5 with Sodium Hydroxide.

### Preparation of Composition C (invention 1)

For the formulation of Composition C, the corresponding amount of Sodium Lauroyl Lactylate was filled into a vessel and melted at 60°C. Afterwards, Lauryl Hydroxysultaine and Cocamidopropyl Hydroxysultaine were filled into the vessel. The mixture was stirred at 60°C until complete homogenization of the surfactants and afterwards sodium citrate was added into the mixture and stirred until complete homogenization. Afterwards, the corresponding amount of water was added, and the pH of the mixture was adjusted to pH=5.5 - 6.5 with sodium hydroxide.

Table 1 summarizes the results for the content of the compositions.

**Table 1:**

| Composition (%wt) | Composition A (Comparative A) | Composition B (Comparative B) | Composition C (Invention 1) |
|---|---|---|---|
| Sodium Lauroyl Lactylate | 10.0 | 10.0 | 10.0 |
| Lauryl Hydroxysultaine | 30.0 | 0.0 | 15.0 |
| Cocamidopropyl Hydroxysultaine | 0.0 | 30.0 | 15.0 |
| Sodium Citrate | 3.0 | 3.0 | 3.0 |
| Sodium Hydroxide | q.s. | q.s. | q.s. |
| deionized water | Up to 100 | Up to 100 | Up to 100 |

Viscosity of the compositions prepared in Example 1 is summarized in Table 2 below. It can be observed that viscosity obtained in Composition C according to the invention is the desired and adequate viscosity for the composition.

Table 2 summarizes the results for viscosity of the composition.

**Table 2:**

| | Composition A (Comparative) | Composition B (Comparative) | Composition C (Invention) |
|---|---|---|---|
| Viscosity at 20°C, cP | 4580 | 185800 | 15700 |

Viscosity of the compositions was determined at 20°C, 24 hours after preparation, with Brookfield viscometer model LV. Composition A viscosity was measured using a spindle number 3 at 6 rpm, for composition B viscosity was measured using a spindle number 4 at 3 rpm and for composition C viscosity was measured using a spindle number 4 at 6 rpm.

### Example 2

### Performance of the compositions

37.5 g of each composition were diluted with deionized water (62.0 g) at room temperature. The formulations were preserved using a commercial preparation of Propanediol and Phenethyl Alcohol and Undecyl Alcohol and Tocopherol (Sensiva PA 30) and the final pH was adjusted to pH=6.5 with sodium hydroxide.

Performance of cosmetic compositions prepared in Example 1 is summarized in Table 3 below. It can be observed that viscosity obtained in Composition C according to the invention is the desired viscosity for the cosmetic composition, showing a viscosity between 5000-15000cP. Additionally, as observed from the foam evaluation, Composition C has a significant better foam speed even in dirty conditions than comparison compositions A and B.

Therefore, by using a combination of amphoteric surfactants, better results can be observed in terms of viscosity and foam than by using each amphoteric surfactant individually.

**Table 3:**

| Properties | Comparative A | Comparative B | Invention 1 |
|---|---|---|---|
| pH (100%) | 6.5 | 6.5 | 6.5 |
| Viscosity at 20°C, cP | 174 | 43200 | 8500 |

| Foam evaluation: | | | |
|---|---|---|---|
| Foam speed at 30 sec (mL) | 424 | 416 | 509 |
| Foam speed(dirty conditions) at 30 sec (mL) | 284 | 274 | 391 |

Viscosity of the compositions was determined at 20°C, 24 hours after preparation, with a Brookfield viscometer model LV. Composition A viscosity was measured using a spindle number 2 at 60 rpm, for composition B viscosity was measured using a spindle number 4 at 3 rpm, and for composition C viscosity was measured using a spindle number 3 at 12rpm.

Foam speed was determined using SITA Foam Tester Analyser R-2000. The samples were prepared at 0.1% a.m. using water hardness of 5°f and pH was adjusted to pH=6.5. The measurement was done at 40°C, stirring a volume of sample of 250 mL during intervals of 10 seconds at 1100 rpm. The foam speed value was taken after 30 seconds of starting the method.

## Claims

1. A composition comprising:
a) at least an acyl lactylate, wherein the acyl lactylate is represented by formula (I) 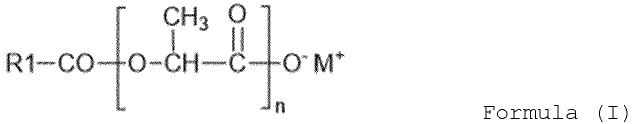 wherein R1 is a linear or branched alkyl or alkenyl group containing from 5 to 23 carbon atoms, preferably from 7 to 17 carbon atoms, more preferably from 9 to 15 carbon atoms, and from 0 to 3 double bonds; n is a number from 1 to 3, preferably 1 to 2; M is an alkali metal, an alkaline earth metal, ammonium, or alkylammonium; preferably an alkali metal, and
b) a mixture of amphoteric surfactants comprising: b1) at least an alkyl or alkenyl hydroxysultaine, wherein b1) is represented by formula (II) 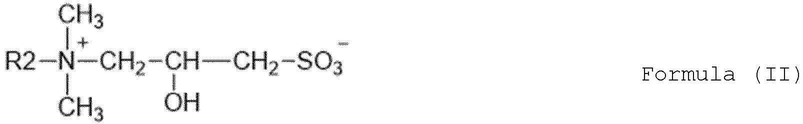
wherein R2 is a linear or branched alkyl or alkenyl group containing from 5 to 23 carbon atoms, preferably from 9 to 17 carbon atoms, more preferably from 9 to 15 carbon atoms, and from 0 to 3 double bonds, and
b2) at least an alkyl or alkenyl amidopropyl hydroxysultaine, wherein b2) is represented by formula (III) 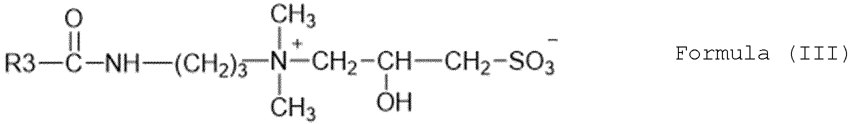
wherein R3 is a linear or branched alkyl or alkenyl group containing from 5 to 23 carbon atoms, preferably from 7 to 21 carbon atoms, more preferably from 7 to 17 carbon atoms, and from 0 to 3 double bonds.

2. The composition according to claim 1, wherein the acyl lactylate is sodium lauroyl lactylate

3. The composition according to claim 1, wherein b1 is lauryl hydroxysultaine and/or wherein b2) is cocamidopropyl hydroxysultaine.

4. The composition according to any one of claims 1 to 3, wherein the ratio by weight of components b1: b2 is from 2:1 to 1:2, preferably from 1.5:1 to 1:1.5, more preferably from 1.2:1 to 1:1.2.

5. The composition according to any one of claims 1 to 4, wherein the amount of component a), component b1) and component b2) is from 10 to 45%wt., preferably from 25 to 45% wt., preferably from 25 to 43% wt., more preferably from 30 to 43% wt., even more preferably from 35 to 42% wt., based on the total amount of the composition, and wherein the composition further comprises water.

6. The composition according to any one of claims 1 to 4, wherein the amount of component a), component b1) and component b2) is from 5 to 30% wt., preferably from 8 to 30% wt., more preferably from 8 up to (but not including) 25% wt., even more preferably from 10 up to (but not including) 25% wt. based on the total amount of the composition., and wherein the composition further comprises water.

7. The composition according to claim 6, wherein the composition further comprises a perfume.

8. A process for producing the composition according to claim 5, comprising the steps:
i) subjecting component a) to a temperature from 50°C to 80°C, preferably at a temperature from 55°C to 65°C; optionally in the presence of water, so that component a) is in liquid form,
ii) mixing component a) and component b1) and b2) at a temperature from 40°C to 60°C; and optionally water, and
iii) stirring to obtain a homogeneous solution,
wherein there is water in step i, ii or both steps.

9. The process according to claim 8, comprising the steps:
0) preparing component a) by reaction of lactic acid with a fatty acid, preferably a fatty acid of formula R1-COOH, wherein R1 is as defined in claim 1, at a temperature from 100°C to 250°C in the presence of an alkali catalyst, such as sodium hydroxide;
i) cooling down of the product obtained in step 0) to a temperature from 50°C to 80°C, preferably at a temperature from 55°C to 65°C; optionally in the presence of water, so that component a) is in liquid form,
ii) mixing component a) and component b1) and b2), and optionally water, at a temperature from 40°C to 60°C;
iii) stirring to obtain a homogeneous solution,
wherein there is water in present in step i), step ii) or both steps.

10. A process for producing the composition according to claim 6, comprising the steps:
1. mixing the composition according to claim 5 with water at room temperature; and
2. stirring to obtain a homogeneous solution,
wherein the total concentration of components a), b1) and b2) in the composition according to claim 5 mixed with water in step 1) is higher than that of the composition obtained after mixing with water.

11. A process according to claim 10, wherein the composition according to claim 5 is prepared by the process defined in claim 8.

12. A process according to claim 10 or 11, wherein the process comprises an additional step of adding a perfume.

13. Use of a composition according to claims 6 or 7, or obtainable by a process according to any one of claims 10 to 12 to prepare a cosmetic composition for the care of skin and/or hair, preferably for cleansing skin and/or hair.

14. Use of a composition according to claims 6 or 7, or obtainable by a process according to any one of claims 10 to 12, for the care of skin and/or hair, preferably for cleansing skin and/or hair.

15. A method to cleanse skin and/or hair comprising the steps of wetting or dampening the skin and/or hair, applying a composition according to claims 6 or 7, or obtainable by a process according to any one of claims 10 to 12, on the skin and/or hair, and rinsing.
